# DEMANDE DE BREVET EUROPEEN

(11) **EP 1 574 224 A1**
(43) Date de publication de la demande: **14.09.2005**
(21) Numéro de dépôt: 04447058.1
(22) Date de dépôt: 10.03.2004
(51) Int. Cl.: A61L 2/26, A61M 5/00

(54) **Boîte de manutention d'objets**

(71) Demandeur: Sybermat, 1348 Louvain-la-Neuve (BE)
(72) Inventeur: Van Roy, Pierre Jacques, 1180 Bruxelles (BE)
(74) Mandataire: Claeys, Pierre

(57) **Abrégé**

Boîte de manutention d'objets (1) comprenant: deux éléments structurels : un corps de boîte (2) et un couvercle (3) de fermeture, présentant entre eux une position d'ouverture et une position de fermeture, des moyens de positionnement d'objets (18) dans la boîte (1) et au moins un élément de verrouillage/déverrouillage (12) dudit couvercle (3), ladite boîte comprenant au moins des moyens réciproques (13a,13b,13c,14a,14b,14c) permettant un déplacement relatif du premier élément structurel par rapport au second élément structurel entre lesdites positions d'ouverture et de fermeture, ledit au moins un élément de verrouillage/déverrouillage (12) étant en position de verrouillage et bloquant les éléments structurels de ladite boîte l'un par rapport à l'autre en position d'ouverture et en position de fermeture de ceux-ci, et des moyens de préhension (6,6') agencés pour une manipulation robotisée.

## Description

La présente invention se rapporte à une boîte de manutention d'objets comprenant:
- deux éléments structurels: un corps de boîte et un couvercle de fermeture du corps de boîte, les deux éléments structurels présentant entre eux une position d'ouverture et une position de fermeture,
- des moyens de positionnement d'objets dans la boîte agencés pour les y bloquer en ordre et en position par fermeture dudit couvercle, et
- au moins un élément de verrouillage/déverrouillage dudit couvercle.
La présente invention se rapporte aussi à un dispositif de manutention d'objets comprenant ladite boîte.

La boîte et le dispositif de manutention peuvent être en particulier, respectivement une boîte et un dispositif pour transporter des seringues à traiter, avant leur remplissage, par exemple, à la vapeur ou avec des produits de stérilisation et pour les amener ensuite à un poste de remplissage.

Une boîte de transport et de manutention de seringues telle qu'indiquée ci-dessus, est connue du document EP1 088 566 qui divulgue une boîte à manipuler par l'homme de métier.

De nos jours, il y a un besoin croissant de sécuriser le traitement de tels objets, en particulier des seringues, et de réduire la manipulation d'objets stériles par un être humain. De plus ces traitements doivent, dans le même ordre d'idée, être rapides et peu coûteux.

Jusqu'à présent, une telle manutention, en particulier pour les seringues, implique une intervention humaine. En effet, un technicien doit prendre la boîte comprenant des rainures de positionnement d'objets sur un chariot de stockage des boîtes de manutention, l'ouvrir, la maintenir ouverte à proximité d'un dispositif automatique d'enfilage de seringues dans la boîte de manutention, attendre la fin du remplissage, fermer la boîte pour bloquer en ordre et en position les seringues, la retourner tout en la maintenant fermée pour que les seringues enfilées soient inversées mais pas renversées pour la stérilisation pour empêcher l'accumulation de produit stérilisant dans le corps de la seringue, la placer dans une autoclave ou un appareillage de stérilisation équivalent, la retourner à nouveau en la maintenant fermée il faut remettre les seringues à l'endroit pour leur remplissage et ensuite l'amener vers un dispositif de remplissage des seringues. C'est ce type de boîte qui est divulgué dans le document susmentionné. Cette boîte est maintenue manuellement en position fermée par l'homme du métier qui la manipule en insérant ses mains dans des rainures présentes sur le couvercle et le corps de la boîte, empêchant de cette manière le déplacement relatif des deux éléments structurels précités.

Toutes les étapes manuelles et toutes les étapes de transport de la boîte et des seringues, de même que les retournements présentent de nombreux risques de contamination par une manipulation manuelle, de renversement du contenu de la boîte, de brûlure de l'homme du métier qui la manipule, de fermeture du couvercle pendant le remplissage, etc.

Dans le but de pallier ces inconvénients, l'invention a pour objet une boîte de manutention permettant une manipulation automatisée, en vue d'éviter les inconvénients précités. La boîte doit de préférence être agencée pour éviter que le contenu de la boîte se répande lors du retournement avant stérilisation et que la boîte se referme pendant le remplissage avec des seringues par exemple. Pour résoudre ces problèmes, il est prévu selon l'invention, une boîte telle que décrite au début, comprenant
- un moyen guidé sur un premier desdits éléments structurels et un moyen de guidage dudit moyen guidé sur un second desdits éléments structurels, le moyen guidé et le moyen de guidage permettant un déplacement relatif du premier élément structurel par rapport au second élément structurel entre lesdites positions d'ouverture et de fermeture,
- des moyens de pivotement réciproques agencés sur lesdits premier et second éléments structurels pour permettre une rotation d'un élément structurel par rapport à l'autre pendant leur déplacement relatif susdit,
- ledit au moins un élément de verrouillage/déverrouillage étant en position de verrouillage et bloquant les éléments structurels de ladite boîte l'un par rapport à l'autre en position d'ouverture et en position de fermeture de ceux-ci, et
- des moyens de préhension agencés pour une manipulation robotisée, les dits moyens guidés et de guidage, lesdits moyens de pivotement, ledit au moins un élément de verrouillage/déverrouillage et les moyens de préhension étant complémentaires d'une commande automatique extérieure permettant leur actionnement et leur déplacement.

D'autres formes de réalisation de la boîte sont indiquées dans les revendications annexées.

Dans une réalisation préférentielle de l'invention, le moyen guidé et le moyen de pivotement réciproque sur ledit premier élément structurel et le moyen de guidage et le moyen de pivotement réciproque sur ledit second élément structurel sont respectivement au moins un élément suiveur et au moins une rainure, ledit élément suiveur étant agencé pour se mouvoir selon une trajectoire définie par ladite rainure. Ceci permet une manipulation plus aisée de la boîte selon l'invention par un dispositif automatisé. En effet, la présence des rainures dans lesquelles les éléments suiveurs sont en mouvement, permet un déplacement reproductible et dès lors aisé à automatiser d'un élément structurel par rapport à l'autre.

Avantageusement, la boîte a une forme parallélépipédique et les éléments structurels comprennent chacun deux parois latérales mutuellement parallèles formant de part et d'autre de la boîte deux paires de parois latérales parallèles.

Dans une réalisation particulièrement avantageuse de l'invention, la boîte de manutention d'objets comprend sur chaque paire de parois latérales des moyens guidés et des moyens de guidage réciproques supplémentaires agencés pour assister le déplacement relatif et limiter la rotation relative du premier élément structurel par rapport au second. Cette caractéristique facilite le verrouillage de la boîte en position ouverte en imposant une position prédéterminée définie par l'ouverture maximale autorisée par les moyens de guidage. La position prédéterminée permet le logement de l'élément suiveur dans un repli de verrouillage de l'élément de verrouillage/déverrouillage.

Dans le cas d'une stérilisation, à la vapeur ou par un autre fluide, d'objets placés dans une boîte de manutention, la présence d'un volume mort, ou la difficulté pour le fluide stérilisant d'accéder en tous les endroits dans la boîte de manutention rallonge souvent le temps nécessaire pour assurer la stérilisation desdits objets. Toute augmentation de la durée d'un procédé ou d'une étape entraîne inévitablement une augmentation des coûts de la manutention. Dans une variante de l'invention, la boîte comprend de nombreuses ouvertures permettant un passage d'un fluide de traitement au travers de la boîte, pour le traitement des objets placés dans ladite boîte par lesdits moyens de positionnement d'objets.

La présente invention est également relative à un dispositif permettant une manipulation automatisée d'une boîte telle qu'indiquée au début, la boîte étant alors manipulée par une pince robotisée. A cette fin, le dispositif selon l'invention comprend:
- une pince de commande pourvue de moyens de préhension, ladite boîte comprenant:

- un moyen guidé sur un premier desdits éléments structurels et un moyen de guidage dudit moyen guidé sur un second desdits éléments structurels, le moyen guidé et le moyen de guidage permettant un déplacement relatif du premier élément structurel par rapport au second élément structurel entre lesdites positions d'ouverture et de fermeture,
- des moyens de pivotement réciproques agencés sur lesdits premier et second éléments structurels pour permettre une rotation d'un élément structurel par rapport à l'autre pendant leur déplacement relatif susdit, ledit au moins un élément de verrouillage/déverrouillage étant en position de verrouillage et bloquant les éléments structurels de ladite boîte l'un par rapport à l'autre en position d'ouverture et en position de fermeture,
- des moyens de préhension complémentaires de ceux de la pince de commande, permettant une prise mutuelle entre la pince et la boîte, et la pince de commande comprend en outre:

- au moins un moyen d'actionnement capable de faire passer ledit au moins un élément de verrouillage/déverrouillage de ladite position de verrouillage à une position de déverrouillage, et éventuellement de sa position de déverrouillage à sa position de verrouillage, lorsque la pince et la boîte sont en position de prise mutuelle, et
- au moins un élément de déplacement capable de déplacer un premier élément structurel de ladite boîte par rapport au second entre les positions d'ouverture et de fermeture de ceux-ci et inversement, lorsque ledit au moins un élément de verrouillage est en position de déverrouillage.

Cette manipulation robotisée de la boîte par la pince de commande diminue les risques de contamination en limitant le contact de la boîte avec l'homme, de plus, la manipulation ouverture, déplacement, retournement et amenée de la boîte par la pince robotisée est une manipulation simple, rapide et peu coûteuse.

D'autres formes de réalisation du dispositif sont indiquées dans les revendications annexées.

D'autres caractéristiques, détails et avantages de l'invention ressortiront de la lecture du mémoire descriptif en faisant référence aux dessins annexés donnés à titre d'exemples non limitatifs.

La figure 1 illustre une vue générale en perspective d'une boîte de manutention d'objets suivant l'invention.

Les figures 2 à 4 sont des vues arrière de la boîte illustrée sur la figure 1, dans différentes positions des moyens de préhension.

Les figures 5 à 7 sont des vues en coupe correspondantes, selon la ligne I-I de la figure 2.

La figure 8 est une vue en coupe de la boîte selon la ligne I-I de la figure 2 illustrant le déverrouillage du dispositif en position fermée.

Les figures 9 et 10 sont des vues en coupe de la boîte selon la ligne I-I de la figure 2 illustrant le dispositif au cours de l'ouverture de la boîte.

La figure 11 est une vue en coupe de la boîte selon la ligne I-I de la figure 2 illustrant le verrouillage de la boîte en position ouverte.

Les figures 12 et 13 sont des vues en coupe de la boîte selon la ligne I-I de la figure 2 illustrant le dispositif au cours de la fermeture de la boîte, avec le déverrouillage de la boîte en position ouverte et le verrouillage de la boîte en position fermée.

Sur les figures, les éléments identiques ou analogues portent les mêmes références.

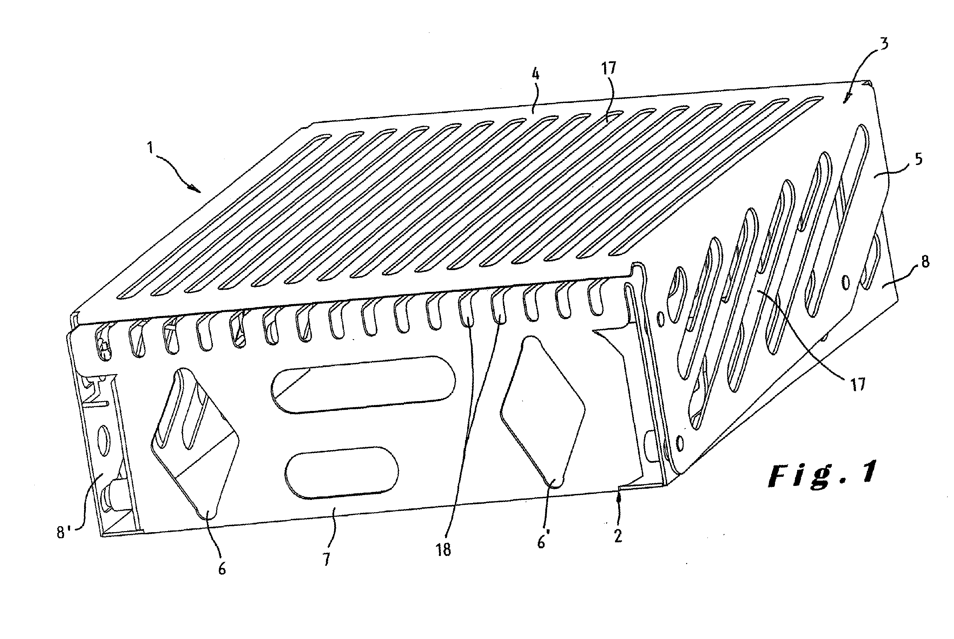

La figure 1 illustre la boîte 1 du dispositif selon l'invention. La boîte 1 comprend deux élément structurels. Dans la réalisation préférentielle donnée ici à titre d'exemple non limitatif, le premier élément structurel 2 représente le corps de la boîte et le second élément structurel 3 représente le couvercle du corps de la boîte, mais il va de soi pour l'homme du métier, que le premier élément structurel peut représenter le couvercle et que le second peut représenter le corps de boîte. Le corps de la boîte est formé de préférence d'une plaque de base, de deux parois frontales 7,7' et de deux parois latérales 8,8'. Le couvercle 3 est formé de préférence à partir d'une tôle d'une pièce, découpée et pliée de manière à présenter une plaque de recouvrement rectangulaire 4 pour le corps de boîte 2 et deux parois latérales 5,5' de couvercle 3. Ces parois latérales 5,5' recouvrent de préférence pratiquement les parois latérales 8,8' du corps de la boîte 2, et forment de part et d'autre de la boîte des paires de parois latérales 5 et 8, 5'et 8'. Le corps de la boîte 2 comprend en outre des moyens de préhension 6,6' situés dans cette réalisation préférentielle sur une paroi frontale arrière 7, du coté opposé à l'ouverture. La boîte comprend de nombreuses ouvertures 17 de passage de fluide de traitement et des moyens de positionnement d'objets 18.

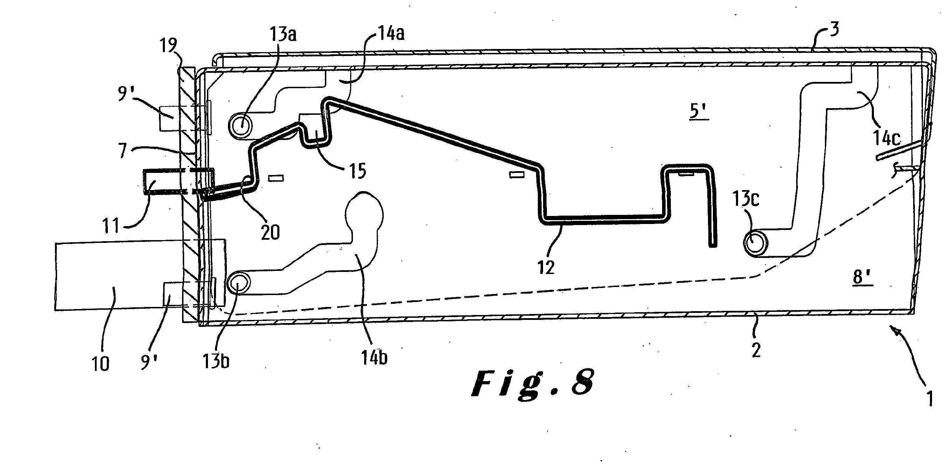

La figure 8 illustre, entre autres, d'une manière schématique, la pince 19 de commande du dispositif selon l'invention. La pince comprend des moyens de préhension 9,9', respectivement complémentaires aux moyens de préhension 6,6' de la boîte 1, des moyens de déplacement 10 et des moyens d'actionnement 11.

La figure 8 illustre aussi la boîte 1 du dispositif selon l'invention. Il ressort de cette figure que la boîte comprend en outre, au moins sur une paire de parois latérales 5 et 8, 5' et 8', un élément de verrouillage/déverrouillage 12, des moyens de pivotement réciproques 13a et 14a, sous la forme respectivement d'un élément suiveur 13a et d'une rainure 14a, des moyens guidés comprenant un élément suiveur 13b et des moyens de guidage comprenant par exemple une rainure 14b. Dans la réalisation préférentielle décrite ici, la boîte comprend aussi un élément suiveur 13c et une rainure 14c supplémentaires. L'élément de verrouillage/déverrouillage comprend au moins un repli de verrouillage 15,20 agencé pour loger l'élément suiveur 13a.

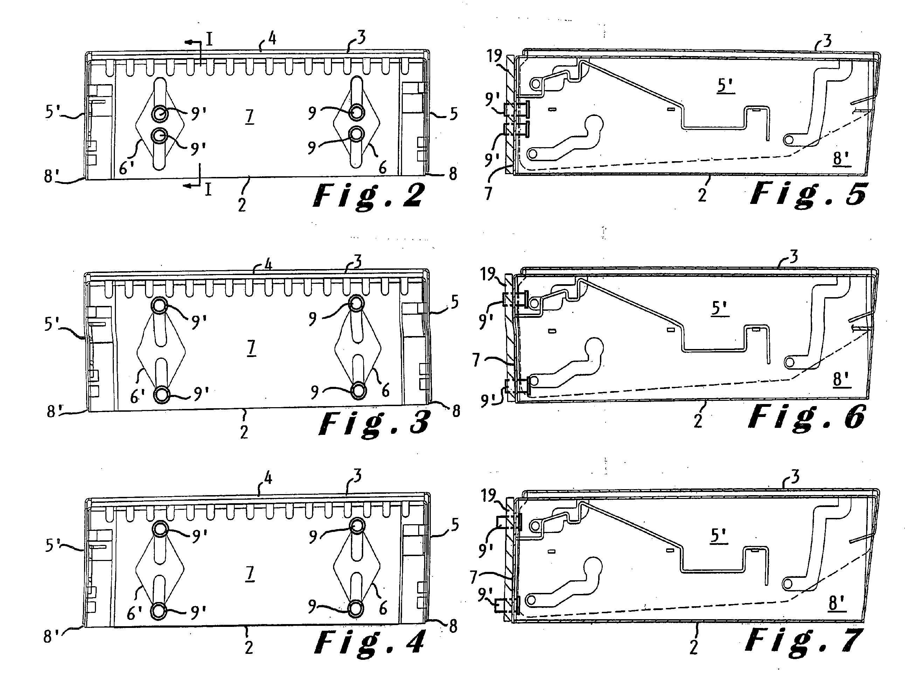

Les figures 2 à 7 représentent les positions des moyens de préhension mutuels (6,6' et 9,9') lors de la prise de la boîte 1 de manutention d'objets par la pince de commande 19. Les figures 2 à 4 sont des vues de face de l'arrière de ladite boîte, les figures 5 à 7 sont des vues en coupe selon la ligne I-I de la figure 2.

Les figures 2 et 5 illustrent la réalisation préférentielle des moyens de préhensions 9,9' de la pince de commande 19 en position d'insertion dans les moyens de préhensions 6,6' de la boîte 1 situés sur la paroi frontale arrière 7. La position d'insertion est obtenue par l'avancée de la pince de commande 19 en position fermée. Les figures 3 et 6 illustrent la positon des moyens de préhension 9,9' de la pince de commande 19, écartés l'un de l'autre, dans la réalisation préférentielle lorsque la pince 19 s'ouvre dans les moyens de préhensions 6,6' de la boîte 1 situés sur la paroi frontale arrière 7. Les moyens de préhension 9,9' de la pince de commande 19 dans cette étape intermédiaire d'ouverture de cette dernière, sont en position d'avancée, comme à l'étape d'insertion. Cette position d'avancée, fermée ou ouverte des moyens de préhension 9,9' de la pince de commande 19 est particulièrement visible aux figures 5 et 6. Les figures 4 et 7 illustrent la position des moyens de préhensions 9,9' de la pince de commande 19 dans la réalisation préférentielle lorsque la pince 19 est ouverte et serrée dans les moyens de préhensions 6,6' de la boîte 1. Les moyens de préhension 9,9' de la pince de commande sont en position de retrait (Fig. 7) pour le serrage des moyens de préhension 9,9' de la pince de commande 19 dans les moyens de préhension 6,6' de la boîte 1. Le serrage mutuels des moyens de préhension 6 et 9, 6' et 9' (Fig. 4 et 7) permet à la pince de commande 19 de manipuler la boîte 1, c'est-à-dire, de la déplacer, de la retourner, de l'incliner, etc. pour autant que la mobilité de la pince de commande 19 le permette.

Les figures 8, 9, 10 et 11 illustrent la séquence des positions relatives des divers moyens et éléments structurels lors de l'ouverture de la boîte, à partir de la position fermée et verrouillée, en passant par le déverrouillage et l'ouverture de cette dernière pour aboutir à l'ouverture et au verrouillage de la boîte 1 dans cette position.

La figure 8 illustre la réalisation préférentielle susmentionnée du dispositif selon l'invention lorsque l'élément de verrouillage/déverrouillage 12 de la boîte 1 et le moyen d'actionnement 11 de la pince 19 sont en position réciproque de déverrouillage de la boîte 1 de sa position fermée.

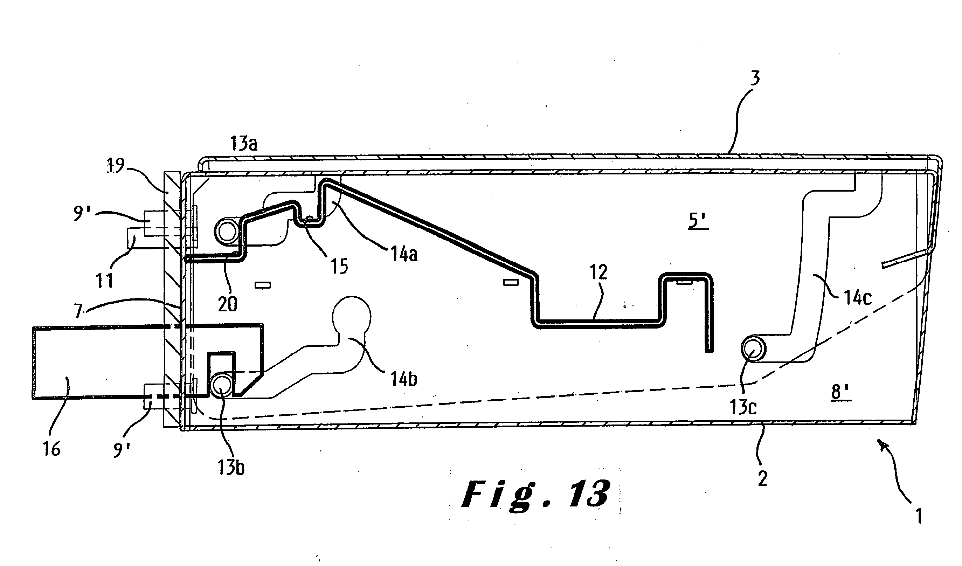

L'avancée du moyen d'actionnement 11 de la pince 19 est réalisée de préférence simultanément avec l'avancée de l'élément de déplacement 10 de la pince de commande 19. Le moyen d'actionnement 11 déverrouille l'élément de verrouillage/déverrouillage 12 de sa position de verrouillage lorsque la boîte 1 est en position fermée. La position de verrouillage en position fermée est illustrée par exemple à la figure 7 mais sera expliquée plus en détails ultérieurement. Lorsque la boîte est en position fermée et verrouillée, l'élément suiveur 13a est bloqué dans sa rainure 14a en une position initiale par un premier repli de verrouillage 20 de l'élément de verrouillage/déverrouillage 12 (voir Fig. 13)

Le moyen d'actionnement 11 de la pince de commande 19 dans la réalisation préférentielle baisse l'élément de verrouillage/déverrouillage 12 au cours de son avancée et libère l'élément suiveur 13a.

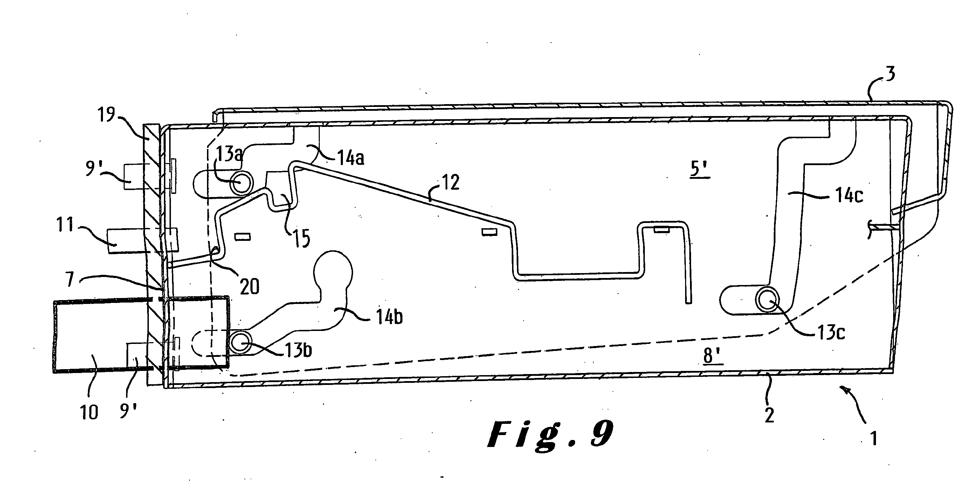

La figure 9 illustre la position réciproque des éléments suiveurs 13a et 13b (constituant dans cette réalisation préférentielle respectivement les moyens réciproques de pivotement et les moyens guidés) dans les rainures 14a et 14b de la boîte 1 de manutention d'objets et l'élément de déplacement 10 de la pince de commande 19 lors de l'ouverture de la boîte 1, celle-ci étant forcément à l'état déverrouillé.

L'ouverture de la boîte 1 est réalisée par une poussée de l'élément de déplacement 10 sur l'élément suiveur 13b positionné dans la réalisation préférentielle sur le couvercle 3 de la boîte 1. Cette poussée de l'élément de déplacement 10 de la pince de commande 19 impose en un premier temps une première translation d'un premier élément structurel par rapport à l'autre. Dans la réalisation préférentielle décrite ici, c'est le couvercle 3 qui subit la translation par rapport au corps 2 de la boîte 1. Les éléments suiveurs 13a et 13b étant tous ici solidaires du couvercle 3, ils subissent tous la même première translation jusqu'à ce que le premier d'entre eux arrive au bout du premier tronçon de sa rainure 14a et 14b. Le premier élément suiveur à finir sa course sur le premier tronçon de sa rainure est, dans la réalisation décrite, l'élément suiveur 13a impliqué dans le verrouillage/déverrouillage de la boîte 1. Il faut noter que cet élément suiveur 13a glisse dans sa rainure 14a au-dessus de l'élément de verrouillage 12, en empêchant ce dernier 12 de remonter dans une position de verrouillage.

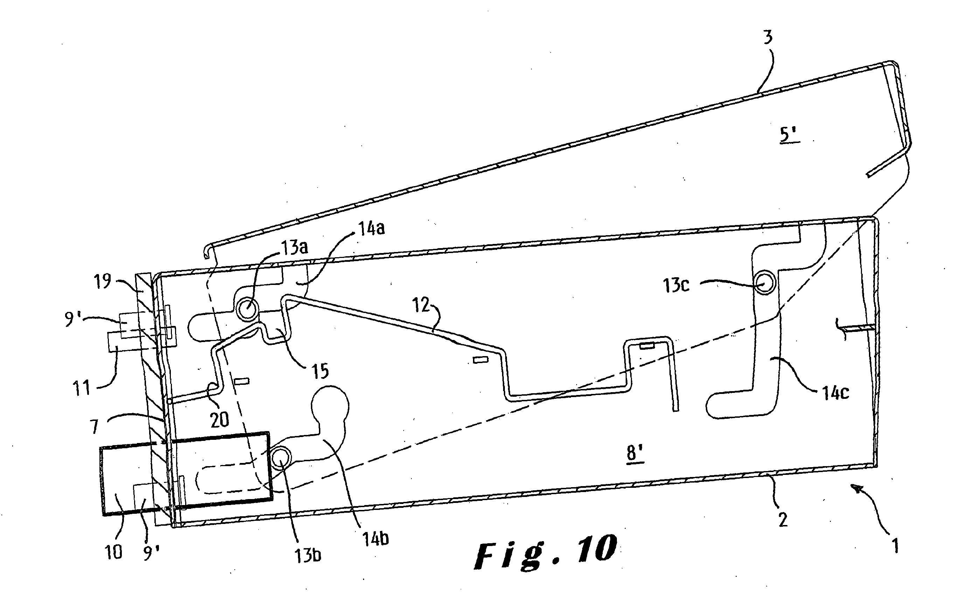

La figure 10 illustre la position réciproque des éléments suiveurs 13a et 13b dans les rainures 14a et 14b de la boîte 1 de manutention d'objets et l'élément de déplacement 10 de la pince de commande 19 pendant l'ouverture de la boîte 1 lors de la rotation du premier élément structurel par rapport au second.

Dans la réalisation préférentielle susmentionnée, puisque l'élément 13a est au bout du premier tronçon de la rainure 14a, et parce que l'élément de déplacement 10 continue sa poussée , l'élément suiveur 13b entame son déplacement dans le deuxième tronçon de sa rainure 14b. Ce deuxième tronçon est un tronçon incliné par rapport au premier tronçon et impose une élévation du couvercle 3 de la boîte 1 autour de l'élément suiveur 13a. La rainure 14a, comprend, quant à elle un second tronçon substantiellement perpendiculaire à son premier tronçon, dès lors puisque les éléments suiveurs 13a,13b sont tous solidaires du couvercle, l'élément suiveur 13a va pousser sur sa rainure 14a et pivoter à cause de l'inclinaison du tronçon oblique de la rainure 14b et le couvercle (premier élément structurel) 3 subit une rotation. Cette rotation est donc due à la combinaison de la géométrie des deux rainures 14a et 14b. L'élément d'actionnement 11 reprend alors la position initiale qu'il avait avant le déverrouillage. La géométrie des trois rainures 14a,14b,14c et particulièrement la géométrie des rainures 14 b et 14 c empêche, par exemple dans le cas d'une manipulation simple, tel qu'un retournement de ladite boîte de manutention (1), que le mouvement de rotation ou que tout autre mouvement de translation dans une direction substantiellement différente de celle imposée par le premier tronçon ait lieu avant le mouvement de translation imposé par ce premier tronçon. Dès lors, la boîte ne peut s'ouvrir lorsque les éléments structurels sont en position de fermeture grâce à l'élément de verrouillage/déverrouillage 12 et/ou grâce à la géométrie des rainures 14a,14b,14c.

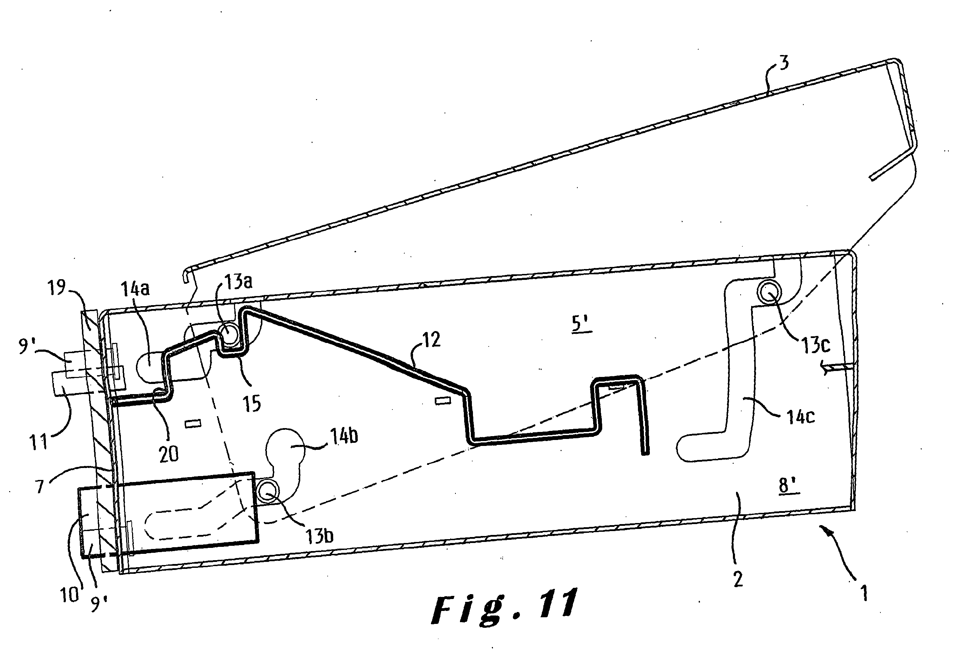

La figure 11 illustre le verrouillage de la boîte 1 en position ouverte. L'inclinaison du deuxième tronçon de la rainure inférieure 14b a permis à l'élément suiveur 13a de passer le deuxième tronçon de sa rainure 14a. Dans la réalisation préférentielle décrite ici, une troisième rainure 14c permet de limiter par sa géométrie l'ouverture maximale du couvercle 3 de la boîte 1, et permet d'imposer une position prédéterminée d'ouverture pour faciliter le verrouillage de la boîte 1 dans cette position d'ouverture. L'élément suiveur 13c arrive au bout du deuxième tronçon de sa rainure 14c en même temps que l'élément suiveur 13b arrive au bout du second tronçon de sa rainure 14b et ceci termine le mouvement de rotation du couvercle 3 par rapport au corps de la boîte 1, l'élément suiveur 13a étant un moyen de pivotement. Comme l'élément de déplacement 10 continue sa poussée, un second mouvement de translation est imposé, jusqu'à ce que l'élément de verrouillage 12, dont la remontée était empêchée par l'élément suiveur 13a, puisse remonter, l'élément suiveur 13a se logeant dans un deuxième repli de verrouillage 15 prévu sur l'élément de verrouillage/déverrouillage 12. Cela empêche la poursuite de deuxième la translation imposée par l'élément de déplacement 10 de la pince de commande 19. La séquence de ces événements provoque le verrouillage de la boîte 1 en position ouverte, le deuxième repli de verrouillage 15 empêchant l'élément suiveur 13a de continuer sa deuxième translation.

Les figures 11, 12 et 13 illustrent la séquence des positions relatives des divers moyens et éléments structurels lors de la fermeture de la boîte, à partir de la position ouverte et verrouillée, en passant par le déverrouillage et la fermeture de cette dernière pour aboutir à la fermeture et au verrouillage de la boîte dans cette position.

La figure 11 a été décrite ci-dessus et représente la boîte en position ouverte et verrouillée. Elle sert de point de départ à l'explication du mécanisme de fermeture de la boîte selon l'invention. La position finale de l'élément suiveur 13a est la position qu'il occupe au moment où la boîte est verrouillée en position ouverte.

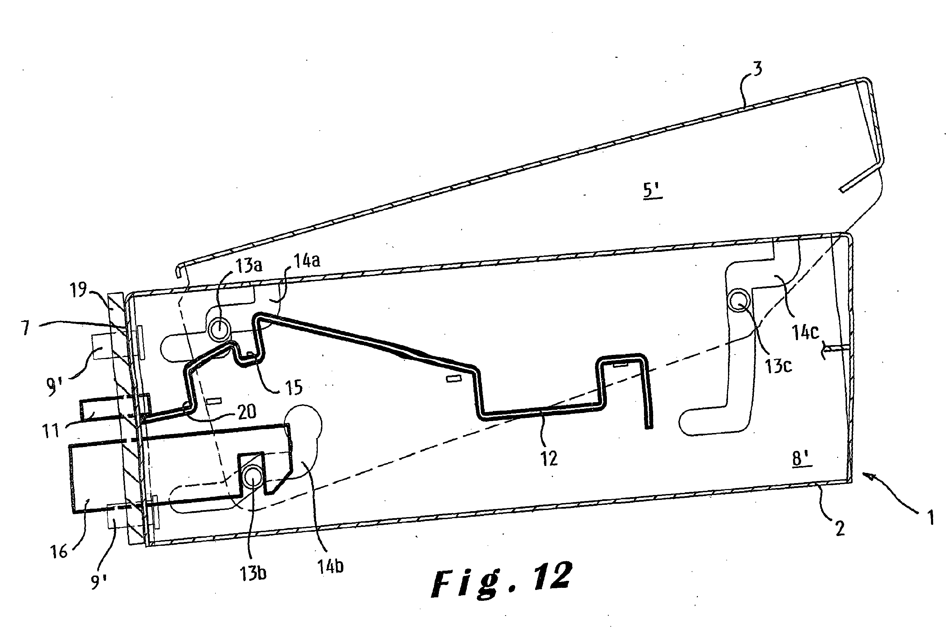

La figure 12 illustre le déverrouillage de la boîte 1 de sa position ouverte et la fermeture de cette dernière. Pour la fermeture de la boîte 1, le moyen d'actionnement 11 de la pince de commande 19 déverrouille l'élément de verrouillage/déverrouillage 12 de la boîte 1 en le poussant vers le bas. Le déverrouillage de l'élément de verrouillage/déverrouillage 12 permet la libération de l'élément suiveur 13a de sa position finale. Au moment où le moyen d'actionnement 11 de la pince de commande 19 déverrouille l'élément de verrouillage/déverrouillage 12 de la boîte 1, l'élément de déplacement impliqué dans la fermeture commence son action. Il peut s'agir des mêmes moyens impliqués dans la poussée de l'élément suiveur 13b (le doigt de poussée 10) ou d'autres moyens. Si les moyens de déplacement impliqués dans l'ouverture et dans la fermeture sont respectivement des moyens de poussée et des moyens de traction, il est alors utile de prévoir un mouvement de retrait des moyens de poussée lorsque la boîte est en position ouverte et verrouillée et avant la fermeture d'amener les moyens de traction.

Dans la réalisation préférentielle donnée ici à titre d'exemple non limitatif, l'élément de déplacement pour l'ouverture est un doigt de poussée 10, tandis que l'élément de déplacement impliqué à la fermeture est un doigt de traction 16. Une fois l'élément de verrouillage/déverrouillage 12 déverrouillé, l'élément suiveur 13a libéré, et une fois l'élément de traction 16 positionné, la traction exercée sur l'élément suiveur 13b impose au couvercle de la boîte de subir d'abord la première translation, la rotation et la seconde translation, ces trois déplacements étant les déplacements inverses par rapport à ceux de l'ouverture. Lors de la traction du couvercle 3 par la pince de commande 19, le moyen d'actionnement 11 de l'élément de verrouillage/déverrouillage 12 reprend sa position initiale. Le déplacement de l'élément suiveur 13a empêche l'élément de verrouillage/déverrouillage 12 de reprendre sa position de verrouillage.

La figure 13 illustre le dispositif selon l'invention en position de fermeture. La position relative des éléments structurels couvercle 3 et corps de boîte 2 étant la même qu'à la figure 7. Une fois que la traction du couvercle 3 par les moyens de traction 16 de la pince de commande 19 est terminée, l'élément suiveur 13a n'empêche plus l'élément de verrouillage/déverrouillage 12 de remonter en position de verrouillage et ce dernier reprend sa position de verrouillage. La boîte 1 est dès lors en position de fermeture et verrouillée.

Il est bien entendu que la présente invention n'est en aucune façon limitée aux formes de réalisations décrites ci-dessus et que bien des modifications peuvent y être apportées sans sortir du cadre des revendications annexées.

## Revendications

1. Boîte de manutention d'objets (1) comprenant:
- deux éléments structurels: un corps de boîte (2) et un couvercle (3) de fermeture du corps de boîte (2), les deux éléments structurels présentant entre eux une position d'ouverture et une position de fermeture,
- des moyens de positionnement d'objets (18) dans la boîte (1) agencés pour les y bloquer en ordre et en position par fermeture dudit couvercle (3), et
- au moins un élément de verrouillage/déverrouillage (12) dudit couvercle (3), **caractérisée en ce qu'**elle comprend en outre
- un moyen guidé (13a,13b,13c,) sur un premier desdits éléments structurels et un moyen de guidage (14a,14b,14c) dudit moyen guidé (13a,13b,13c) sur un second desdits éléments structurels, le moyen guidé (13a,13b,13c) et le moyen de guidage (14a,14b,14c) permettant un déplacement relatif du premier élément structurel par rapport au second élément structurel entre lesdites positions d'ouverture et de fermeture,
- des moyens de pivotement (13a et 14a) réciproques agencés sur lesdits premier et second éléments structurels pour permettre une rotation d'un élément structurel par rapport à l'autre pendant leur déplacement relatif susdit,
- ledit au moins un élément de verrouillage/déverrouillage (12) étant en position de verrouillage et bloquant les éléments structurels de ladite boîte l'un par rapport à l'autre en position d'ouverture et en position de fermeture de ceux-ci, et
- des moyens de préhension (6,6') agencés pour une manipulation robotisée,
les dits moyens guidés (13a,13b,13c) et de guidage (14a,14b,14c), lesdits moyens de pivotement (13a,13b,13c et 14a,14b,14c), ledit au moins un élément de verrouillage/déverrouillage (12) et les moyens de préhension (6,6') étant complémentaires d'une commande automatique extérieure permettant leur actionnement et leur déplacement,

2. Boîte de manutention d'objets (1) selon la revendication 1, **caractérisée en ce que** la boîte (1) a une forme parallélépipédique et **en ce que** les éléments structurels comprennent chacun deux parois latérales (5,5' et 8,8') mutuellement parallèles formant de part et d'autre de la boîte deux paires de parois latérales parallèles (5 et 8, 5'et 8'), les moyens guidés (13a,13b,13c) et de guidage (14a,14b,14c) et les moyens de pivotement réciproques (13a et 14a) étant situés sur au moins une desdites paires de parois latérales (5 et 8, 5' et 8').

3. Boîte de manutention d'objets (1) selon l'une des revendications 1 ou 2, **caractérisée en ce que** le moyen guidé (13a,13b,13) et le moyen de pivotement réciproque (13a) sur ledit premier élément structurel et le moyen de guidage (14a,14b,14c) et le moyen de pivotement réciproque (14a) sur ledit second élément structurel sont respectivement au moins un élément suiveur (13a,13b,13c) et au moins une rainure (14a,14b,14c), ledit élément suiveur (13a,13b,13c) étant agencé pour se mouvoir selon une trajectoire définie par ladite rainure (14a,14b,14c).

4. Boîte de manutention d'objets (1) selon l'une des revendications 1 à 3, **caractérisée en ce que** ladite au moins une rainure (14a,14b,14c) présente plusieurs tronçons successifs d'orientations différentes de façon à obtenir successivement au moins une translation, une rotation et au moins une translation supplémentaire du premier élément structurel par rapport au second.

5. Boîte de manutention d'objets (1) selon l'une des revendications 3 ou 4, **caractérisée en ce** chaque paire de parois latérales (5 et 8, 5' et 8') présentent plusieurs rainures séparées (14a,14b,14c), lesdites rainures (14a,14b,14c) présentant une géométrie réciproquement différente.

6. Boîte de manutention d'objets (1) selon l'une des revendications 1 à 5, **caractérisée en ce que** ledit au moins un moyen de verrouillage/déverrouillage (12) comprend au moins un repli de verrouillage (15,20) agencé pour y loger un moyen guidé (13a) et empêcher le déplacement relatif dudit premier élément structurel par rapport audit second, dans leur position d'ouverture et de fermeture.

7. Boîte de manutention d'objets (1) selon l'une des revendications 2 à 6, **caractérisée en ce que** chaque paire de parois latérales (5 et 8, 5'et 8') comprend en outre des moyens guidés (13c) et des moyens de guidage réciproques (14c) supplémentaires agencés pour assister ledit déplacement relatif et limiter la rotation relative du premier élément structurel par rapport au second.

8. Boîte de manutention d'objets (1) selon l'une des revendications 1 à 7, **caractérisée en ce que** la boîte (1) comprend de nombreuses ouvertures (17) permettant un passage d'un fluide de traitement au travers de la boîte, pour le traitement des objets placés dans ladite boîte par lesdits moyens de positionnement (18).

9. Dispositif de manutention d'objets comprenant:
- une boîte (1) comportant:
- deux éléments structurels: un corps de boîte (2) et un couvercle (3) de fermeture du corps de boîte (2), les deux éléments structurels présentant entre eux une position d'ouverture et une position de fermeture,
- des moyens de positionnement d'objets dans la boîte (1) agencés pour les y bloquer en ordre et en position par fermeture dudit couvercle, et
- au moins un élément de verrouillage/déverrouillage (12) dudit couvercle (3),
**caractérisé en ce que** ledit dispositif comprend
- une pince de commande (19) pourvue de moyens de préhension (9,9'), **en ce que** ladite boîte (1) comprend en outre:
- un moyen guidé (13a,13b,13c) sur un premier desdits éléments structurels et un moyen de guidage (14a,14b,14c) dudit moyen guidé sur un second desdits éléments structurels, le moyen guidé (13a,13b,13c) et le moyen de guidage (14a,14a,14b) permettant un déplacement relatif du premier élément structurel par rapport au second élément structurel entre lesdites positions d'ouverture et de fermeture,
- des moyens de pivotement réciproques (13a et 14a) agencés sur lesdits premier et second éléments structurels pour permettre une rotation d'un élément structurel par rapport à l'autre pendant leur déplacement relatif susdit,
ledit au moins un élément de verrouillage/déverrouillage (12) étant en position de verrouillage et bloquant les éléments structurels de ladite boîte (1) l'un par rapport à l'autre en position d'ouverture et en position de fermeture,
- des moyens de préhension (6,6') complémentaires de ceux de la pince de commande (19), permettant une prise mutuelle entre la pince (19) et la boîte (1), et
**en ce que** la pince de commande (19) comprend
- au moins un moyen d'actionnement (11) capable de faire passer ledit au moins un élément de verrouillage/déverrouillage (12) de ladite position de verrouillage à une position de déverrouillage, et éventuellement de sa position de déverrouillage à sa position de verrouillage, lorsque la pince (19) et la boîte (1) sont en position de prise mutuelle, et
- au moins un élément de déplacement (10) capable de déplacer un premier élément structurel de ladite boîte par rapport au second entre les positions d'ouverture et de fermeture de ceux-ci et inversement, lorsque ledit au moins un élément de verrouillage (12) est en position de déverrouillage.

10. Dispositif de manutention d'objets selon la revendication 9, **caractérisé en ce que** la boîte (1) a une forme parallélépipédique et **en ce que** les deux parois latérales mutuellement parallèles (5,5' et 8,8') formant de part et d'autre de la boîte deux paires de parois latérales parallèles (5 et 8, 5' et 8'), les moyens guidés (13a,13b,13c) et de guidage (14a,14b,14c) et les moyens de pivotement réciproques (13a et 14a) étant situés sur au moins une desdites paires de parois latérales (5 et 8, 5' et 8').

11. Dispositif de manutention d'objets selon l'une des revendications 9 ou 10, **caractérisé en ce que** le moyen guidé (13,13b,13c) et le moyen de pivotement réciproque (13a) sur ledit premier élément structurel et le moyen de guidage (14a,14b,14c) et le moyen de pivotement réciproque (14a) sur ledit second élément structurel sont respectivement au moins un élément suiveur (13a,13b,13c) et au moins une rainure (14a,14b,14c), ledit élément suiveur (13a,13b,13c) étant agencé pour se mouvoir selon une trajectoire définie par ladite rainure (14a,14b,14c), ladite rainure (14a,14b,14c) présentant plusieurs tronçons successifs d'orientations différentes de façon à obtenir un déplacement en translation, une rotation et au moins une translation supplémentaire du premier élément structurel par rapport au second.

12. Dispositif de manutention d'objets selon l'une des revendications 9 à 11, **caractérisé en ce que** ledit au moins un moyen de verrouillage/déverrouillage (12) comprend au moins un repli de verrouillage (15,20) agencé pour loger un élément suiveur (13a,13b,13c) et empêcher le mouvement relatif dudit premier élément structurel par rapport audit second.

13. Dispositif de manutention d'objets selon l'une des revendications 9 à 12, **caractérisé en ce que** les moyens de pivotement (13a et 14a) et les moyens guidés (13a,13b,13c) et de guidage (14a,14b,14c) consistent en plusieurs éléments suiveurs (13a,13b,13c) et plusieurs rainures séparés (14a,14b,14c), lesdites rainures (14a,14b,14c) de géométrie différente, disposées sur chacune desdites paires de parois latérales (5 et 8,5' et 8').

14. Dispositif de manutention d'objets selon l'une des revendications 9 à 13, **caractérisé en ce que** chaque paroi latérale (5,5' et 8,8') comprend en outre des moyens guidés (13c) et des moyens de guidage (14c) réciproques supplémentaires agencés pour soutenir le déplacement relatif et limiter la rotation relative du premier élément structurel par rapport au second.

15. Dispositif de manutention d'objets selon l'une des revendications 9 à 14, **caractérisé en ce que** la boîte (1) comprend de nombreuses ouvertures (17) permettant un passage d'un fluide de traitement au travers de la boîte (1), pour le traitement des objets placés dans ladite boîte par lesdits moyens de positionnement (18).

16. Dispositif de manutention d'objets selon l'une des revendications 8 à 15, **caractérisé en ce qu'**un élément de déplacement (10) susdit de ladite pince (19) est un doigt de poussée (10) agencé pour pousser un élément suiveur (13b) servant de moyen guidé (13a,13b,13c) de ladite boîte (1) dans une direction substantiellement parallèle à la trajectoire définie par une rainure (14b) servant de moyen de guidage (14a,14b,14c) de ladite boîte et pour déplacer ledit premier élément structurel par rapport au second.

17. Dispositif de manutention d'objets selon l'une des revendications 9 à 16, **caractérisé en ce qu'**un élément de déplacement (10) susdit de ladite pince (19) est un doigt de traction (16) agencé pour tirer un élément guidé (13b) de ladite boîte (1) dans une direction substantiellement parallèle à la trajectoire définie par une rainure de guidage (14b) de ladite boîte (1) et pour déplacer ledit premier élément structurel par rapport au second.

18. Dispositif de manutention d'objets selon l'une des revendications 9 à 17, **caractérisé en ce que** lesdits moyens de préhension (6,6', 9,9') mutuels entre ladite boîte (1) et ladite pince (19) étant disposés sur un desdits deux éléments structurels (2 ou 3), l'autre desdits deux éléments structurels (3 ou 2) est celui qui subit le déplacement et la rotation.

19. Dispositif de manutention d'objets selon l'une des revendications 9 à 18, **caractérisé en ce que** lesdits moyens de préhension mutuels (9,9' , 6,6') entre ladite boîte (1) et ladite pince (19) étant disposés au niveau de la boîte (1) sur le second élément structurel, le premier élément structurel étant celui qui subit le déplacement et la rotation.

20. Boîte (1) à mettre en oeuvre dans le dispositif de manutention d'objets selon l'une quelconque des revendications 9 à 19.
